# EUROPEAN PATENT APPLICATION

(11) **EP 4 410 780 A1**
(43) Date of publication of application: **07.08.2024**
(21) Application number: 22876295.1
(22) Date of filing: 28.09.2022
(51) Int. Cl.: C07D 307/68

(54) **METHOD FOR PRODUCING ALKYLFURANCARBOXYLIC ACID ESTER**

(30) Priority: 30.09.2021 JP 2021161519
(71) Applicant: MITSUBISHI GAS CHEMICAL COMPANY, INC., Chiyoda-ku Tokyo 100-8324 (JP)
(72) Inventor: FUJII, Shiki, Tokyo 125-0051 (JP); HARA, Kuniho, Tokyo 125-0051 (JP); OHNO, Daisuke, Tokyo 100-8324 (JP)
(74) Representative: Müller-Boré & Partner Patentanwälte PartG mbB
(86) International application number: PCT/JP2022/036070
(87) International publication number: WO 2023/054429

(57) **Abstract**

To provide a new method for producing an alkylfurancarboxylic acid ester. Provided is a method for producing an alkylfurancarboxylic acid ester, including:
performing an oxidation-reduction reaction between an alkylfuranaldehyde and a compound represented by A-OH (where A is an organic group having 1 to 10 carbon atom(s)) in the presence of a catalyst and a base, and the catalyst is an N-heterocyclic carbene with a pKa of 33.0 or less, the pKa being calculated from a free energy obtained by determining a stable structure of a catalytic molecule in methanol by an SMD method using Gaussian 16.

## Description

### Technical Field

An object of the present invention is to provide a method for producing an alkylfurancarboxylic acid ester. In particular, the present invention relates to a method for producing an alkylfurancarboxylic acid ester from an alkylfuranaldehyde as a starting material.

### Background Art

Methods for producing alkylfurancarboxylic acid esters have been studied. For example, Non-Patent Literature 1 describes production of 5-methyl-2-furoate or the like from 5-methyl-2-furaldehyde.

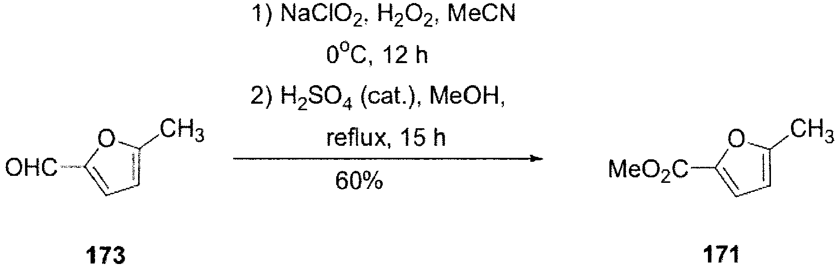

### Citation List

### Non-Patent Literature

[Non-Patent Literature 1] Stereoselective Synthesis of 1, 2-marine Furanoids : Applications towards the Preparation of Cyclopropanecarboxylated Natural Products and Unnatural Amino Acids, University of Regensburg, 2006 Graduation Thesis

### Summary of Invention

### Technical Problem

Although the method described in Non-Patent Document 1 is known, a new method for producing an alkylfurancarboxylic acid ester is required.

Therefore, an object of the present invention is to solve such a problem and provide a new method for producing an alkylfurancarboxylic acid ester.

### Solution to Problem

As a result of studies conducted by the present inventors in view of the aforementioned problem, it has been found that the nucleophilic reaction to the alkylfuranaldehyde can sufficiently proceed by using an N-heterocyclic carbene having a predetermined pKa or less as a catalyst, and the present invention has been completed based on the finding. Specifically, the issues described above are solved by the following solutions.
(1) A method for producing an alkylfurancarboxylic acid ester, including:
   performing an oxidation-reduction reaction between an alkylfuranaldehyde and a compound represented by A-OH (where A is an organic group having 1 to 10 carbon atom(s)) in the presence of a catalyst and a base,
   wherein the catalyst is an N-heterocyclic carbene with a pKa of 33.0 or less, the pKa being calculated from a free energy obtained by determining a stable structure of a catalytic molecule in methanol by an SMD method using Gaussian 16.
(2) The method for producing an alkylfurancarboxylic acid ester according to (1), wherein the method further includes the performing the oxidation-reduction reaction in the presence of an oxidizing agent.
(3) The method for producing an alkylfurancarboxylic acid ester according to (2), wherein the oxidizing agent is quinone.
(4) The method for producing an alkylfurancarboxylic acid ester according to (2), wherein the oxidizing agent comprises at least one oxidizing agent represented by Formula (O): 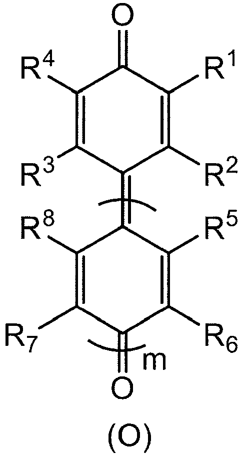 where each of R¹ to R⁸ independently represents a hydrogen atom, an alkyl group having 1 to 12 carbon atom(s), an alkenyl group having 2 to 12 carbon atoms, an alkynyl group having 2 to 12 carbon atoms, an alkoxy group having 1 to 12 carbon atom(s), an aryl group having 6 to 18 carbon atoms, an acyl group having 2 to 12 carbon atoms, or a hydroxy group; in a case where R¹ to R⁸ each are independently an alkyl group, an alkenyl group, an alkynyl group, an alkoxy group, an aryl group, or an acyl group, the groups of R¹ to R⁸ are each independently unsubstituted or substituted with a substituent boned to a carbon atom in the group, the substituent being an alkyl group having 1 to 6 carbon atom(s), an alkoxy group having 1 to 6 carbon atom(s), a hydroxy group, a halogen atom, a cyano group, or a nitro group; m is 1 or 0; and each pair of R¹ and R², R³ and R⁴, R⁵ and R⁶, and R⁷ and R⁸ may be independently bonded or condensed to each other to form a ring.
(5) The method for producing an alkylfurancarboxylic acid ester according to (4), wherein the oxidizing agent represented by Formula (O) includes at least one oxidizing agent represented by Formula (0-3), 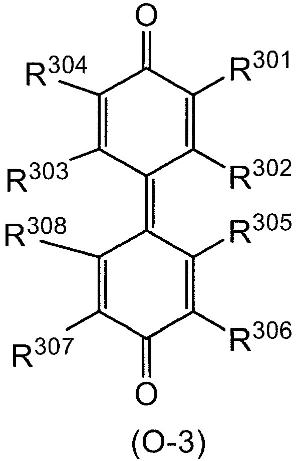 where each of R³⁰¹ to R³⁰⁸ in Formula (O-3) independently represents a group as defined for the R¹ in Formula (O).
(6) The method for producing an alkylfurancarboxylic acid ester according to any one of (1) to (5), wherein the catalyst has a pKa of 16.0 or more.
(7) The method for producing an alkylfurancarboxylic acid ester according to any one of (1) to (6), wherein the catalyst has a pKa of 30.0 or less.
(8) The method for producing an alkylfurancarboxylic acid ester according to any one of (1) to (7), wherein the catalyst includes at least one selected from the group consisting of triazolium, imidazolinium, imidazolium, and thiazolium.
(9) The method for producing an alkylfurancarboxylic acid ester according to any one of (1) to (8), wherein the catalyst includes at least one catalyst represented by the Formula (C) : 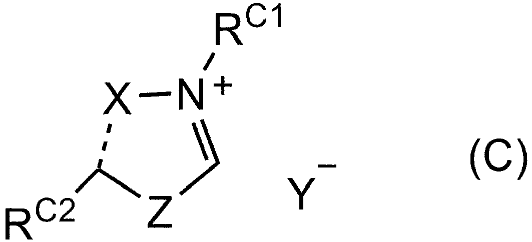 where each of R^{C1} and R^{C2} independently represents a hydrogen atom, an alkyl group having 1 to 12 carbon atom(s), an alkoxy group having 1 to 12 carbon atom(s), an aryl group having 6 to 18 carbon atoms, a heteroaryl group having 4 to 16 carbon atoms, a halogenated alkyl group having 1 to 12 carbon atom(s), a halogenated aryl group having 6 to 18 carbon atoms, an alkylaryl group having 7 to 20 carbon atoms, or an arylalkyl group having 7 to 20 carbon atoms; 2 represents -S- or -NR^{c3}-; X is a methine group (=CR^{C5}-), a nitrogen atom (=N-) or a methylene group (-CR^{C6}₂-); in a case where X is a methine group or a nitrogen atom, the broken line in Formula (C) represents a double bond, and when X is a methylene group, the broken line in Formula (C) represents a single bond; where each of R^{C3}, R^{C5}, and R^{C6} independently represents a hydrogen atom, an alkyl group having 1 to 12 carbon atom(s), an alkenyl group having 2 to 12 carbon atoms, an alkynyl group having 2 to 12 carbon atoms, an aryl group having 6 to 18 carbon atoms, an alkoxy group having 1 to 12 carbon atom(s), an acyl group having 2 to 12 carbon atoms, a hydroxy group, a carboxy group, or a halogen atom; Y⁻ represents a counter anion; and each pair of R^{C2} and R^{C3}, R^{C2} and R^{C5}, R^{C2} and R^{C6} may be independently bonded or condensed with each other to form a ring.
(10) The method for producing an alkylfurancarboxylic acid ester according to (9), wherein the catalyst represented by Formula (C) includes at least one of catalysts represented by Formula (C2) to Formula (C6): 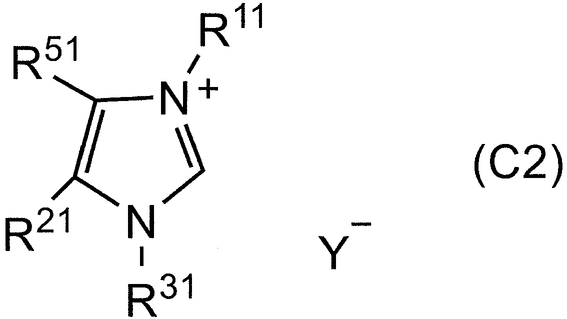
   where, in Formula (C2), R¹¹ represents a group as defined for the R^{C1}, R²¹ represents a group as defined for the R^{C2}, R³¹ represents a group as defined for the R^{C3}, R⁵¹ represents a group as defined for the R^{C5}, Y⁻ represents a counter anion, and each pair of R²¹ and R³¹, R²¹ and R⁵¹, and R¹¹ and R⁵¹ may be independently bonded or condensed with each other to form a ring; 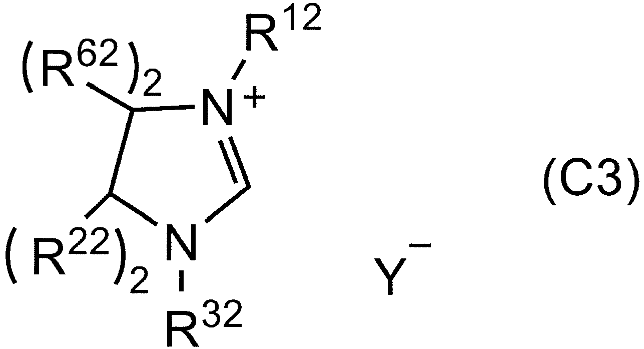
   where, in Formula (C3), R¹² represents a group as defined for the R^{C1}, R²² represents a group as defined for the R^{C2}, R³² represents a group as defined for the R^{C3}, R⁶² represents a group as defined for the R^{C6}, Y⁻ represents a counter anion, and each pair of R²² and R³², R²² and R⁶², and R¹² and R⁶² may be independently bonded or condensed with each other to form a ring; 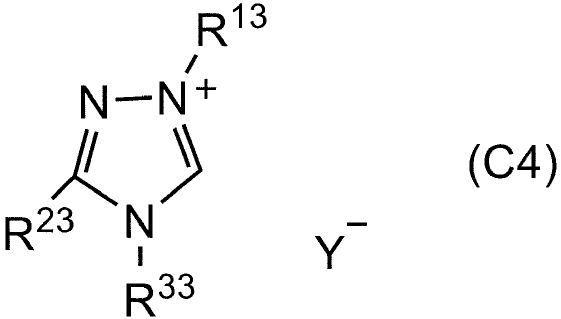
   where, in Formula (C4), R¹³ represents a group as defined for the R^{C1}, R²³ represents a group as defined for the R^{C2}, R³³ represents a group as defined for the R^{C3}, Y⁻ represents a counter anion, and R²³ and R³³ may be bonded or condensed to each other to form a ring; 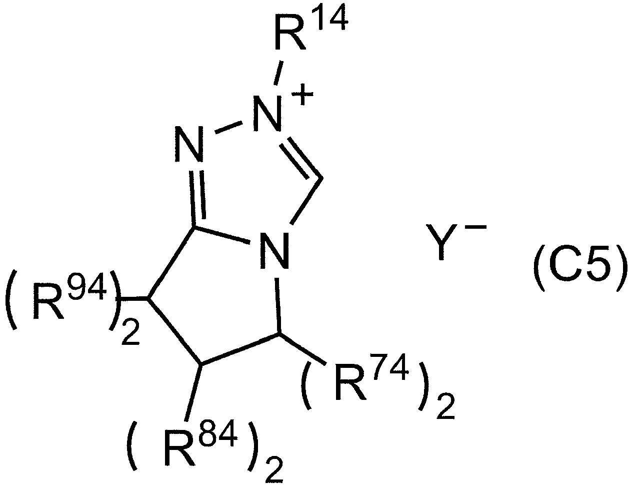
   where, in Formula (C5), R¹⁴ represents a group as defined for the R^{C1}, R⁷⁴, R⁸⁴, and R⁹⁴ each independently represent a group as defined for the R^{C2}, Y⁻ represents a counter anion; 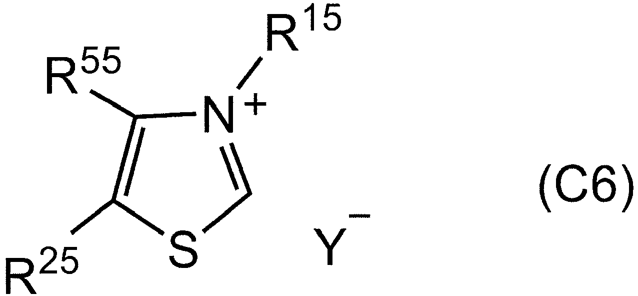
   where, in Formula (C6), R¹⁵ represents a group as defined for the R^{C1}, R²⁵ represents a group as defined for the R^{C2}, R⁵⁵ represents a group as defined for the R^{C5}, Y⁻ represents a counter anion, and each pair of R¹⁵ and R⁵⁵, and R⁵⁵ and R²⁵ may be independently bonded or condensed to each other to form a ring.
(11) The method for producing an alkylfurancarboxylic acid ester according to any one of (1) to (10), wherein the alkylfuranaldehyde is a compound represented by Formula (F1): 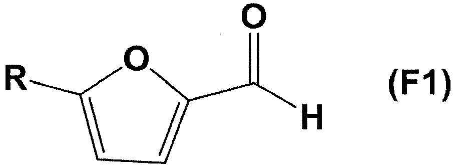 where, in Formula (F1), R is an alkyl group having from 1 to 10 carbon atom(s).
(12) The method for producing an alkylfurancarboxylic acid ester according to any one of (1) to (11), wherein the alkylfurancarboxylic acid ester is a compound represented by Formula (F2): 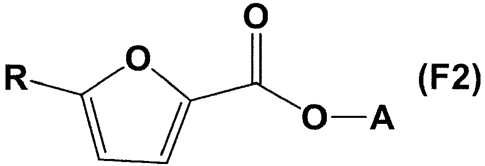 where, in Formula (F2), R is an alkyl group having 1 to 10 carbon atom(s), and A is an organic group having 1 to 10 carbon atom(s).
(13) The method for producing an alkylfurancarboxylic acid ester according to any one of (1) to (12), wherein, in the compound represented by A-OH, A is an alkyl group having 1 to 5 carbon atom(s).

### Advantageous Effects of Invention

According to the present invention, a new method for producing an alkylfurancarboxylic acid ester can be provided.

### Description of Embodiments

Hereinafter, embodiments for conducting the present invention (referred to simply as "the present embodiments" below) will be described in detail. Note that the following embodiments are examples for describing the present invention, and the present invention is not limited to these embodiments.

In the present specification, "from ... to ..." or "of ... to ..." is used to mean that the numerical values described before and after "to" are included as the lower limit and the upper limit, respectively.

In the present specification, various physical property values and characteristic values are values at 23°C unless otherwise noted.

In a description of a group (atomic group) in the present specification, a description not specifying whether the group is a substituted group or an unsubstituted group is meant to include a group (atomic group) having a substituent as well as a group (atomic group) having no substituent. For example, an "alkyl group" includes not only an alkyl group having no substituent (unsubstituted alkyl group) but also an alkyl group having a substituent (substituted alkyl group). In the present specification, a description not specifying whether the group is a substituted group or an unsubstituted group means that the group is preferably an unsubstituted group.

When a measurement method or the like of a standard set forth in the present specification differs depending on the year, it is based on the standard as of January 1, 2021 unless otherwise stated.

pKa means an acid dissociation constant.

A method for producing alkylfurancarboxylic acid ester according to the present embodiment includes performing an oxidation-reduction reaction between an alkylfuranaldehyde and a compound represented by A-OH (where A is an organic group having 1 to 10 carbon atom(s)) in the presence of a catalyst and a base, and the catalyst is an N-heterocyclic carbene with a pKa of 33.0 or less, the pKa being calculated from a free energy obtained by determining a stable structure of a catalytic molecule in methanol by an SMD method using Gaussian 16.

With such a configuration, a novel method for producing an alkylfurancarboxylic acid ester can be provided.

Furthermore, according to the present embodiment, the oxidation-reduction reaction between the alkylfuranaldehyde and the compound represented by A-OH (where A is an organic group having 1 to 10 carbon atom(s)), which is an intermolecular oxidation-reduction reaction, can proceed effectively to produce an alkylfurancarboxylic acid ester at a high yield. The alkylfuranaldehyde has an advantage in that a salt is not formed as a by-product because the oxidation-reduction reaction can proceed intermolecularly. In addition, according to the present embodiment, an alkylcarboxylic acid ester can be produced by a one step reaction.

In the production method of the present embodiment, the alkylfuranaldehyde serves as a substrate for the oxidation-reduction reaction, and the alkylfuranaldehyde is usually added to the reaction system. However, the alkylfuranaldehyde may also be an intermediate for producing the alkylfurancarboxylic acid ester.

The alkylfuranaldehyde is a furan having an alkyl group and an aldehyde group as substituents, and may have the other substituent. Examples of the other substituent include an alkyl group having 1 to 6 carbon atom(s), an alkoxy group having 1 to 6 carbon atom(s), a hydroxy group, a halogen atom, a cyano group, and a nitro group. It is preferable that the alkylfuranaldehyde has no substituent other than an alkyl group and an aldehyde group.

The number of carbon atoms of the alkyl group having 1 to 10 carbon atom(s) contained in the alkylfuranaldehyde is preferably 8 or less, more preferably 6 or less, even more preferably 5 or less, even more preferably 4 or less, even more preferably 3 or less, may be 2 or less, or may be 1.

The molecular weight of the alkylfuranaldehyde is preferably in a range of 111 to 500, more preferably in a range of 111 to 300.

The alkylfuranaldehyde may be preferably a compound represented by Formula (F1): 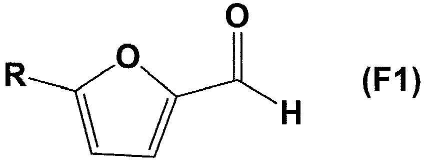 where, in Formula (F1), R is an alkyl group having from 1 to 10 carbon atom(s).

The number of carbon atoms of the alkyl group as R is preferably 8 or less, more preferably 6 or less, even more preferably 5 or less, even more preferably 4 or less, even more preferably 3 or less, may be 2 or less, or may be 1.

Specific examples of R include a methyl group, an ethyl group, an isopropyl group, an n-propyl group, an isobutyl group, an n-butyl group and a tert-butyl group. A methyl group, an ethyl group and an isopropyl group are preferable, and a methyl group is more preferable.

In the present embodiment, the alkylfuranaldehyde may include a single type of alkylfuranaldehyde, or two or more types of alkylfuranaldehydes.

The production method of the present embodiment aims to produce an alkylfurancarboxylic acid ester. Usually, the produced alkylfurancarboxylic acid ester (product mixture) is taken out from the reaction system and, if necessary, the catalyst is separated therefrom or impurities (by-products) are removed therefrom. However, the alkylfurancarboxylic acid ester may also be an intermediate for the preparation of a different compound. That is, the subsequent reaction may be allowed to proceed further in the same reaction system.

The alkyl group (the alkyl group corresponding to R in Formula (F2)) contained in the alkylfurancarboxylic acid ester is the same as the alkyl group contained in the alkylfuranaldehyde (the alkyl group corresponding to R in Formula (F1)).

The alkylfurancarboxylic acid ester may be preferably a compound represented by Formula (F2): 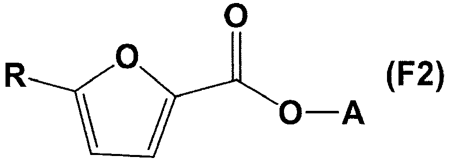 where, in Formula (F2), R is an alkyl group having 1 to 10 carbon atom(s), and A is an organic group having 1 to 10 carbon atom(s).

R is as defined for the R in Formula (F1). A is as defined for the A in the compound represented by A-OH.

In the production method of the present embodiment, an oxidation-reduction reaction is performed between an alkylfuranaldehyde and a compound represented by A-OH (where A is an organic group having 1 to 10 carbon atom(s)). That is, the compound represented by A-OH acts as a nucleophilic agent for a nucleophilic reaction with the formyl group of the alkylfuranaldehyde.

The organic group having 1 to 10 carbon atom(s) which is A in A-OH is preferably a hydrocarbon group having 1 to 10 carbon atom(s) or a hydrocarbon group having 1 to 10 carbon atom(s) with -O- and/or -C(=O)-, more preferably an alkyl group having 1 to 10 carbon atom(s) or an alkyl group having 1 to 10 carbon atom(s) with -O- and/or -C(=O)-, and preferably an alkyl group having 1 to 10 carbon atom(s).

The number of carbon atoms in A may be preferably 8 or less, more preferably 6 or less, even more preferably 5 or less, even more preferably 4 or less, even more preferably 3 or less, may be 2 or less, or may be 1.

The hydrocarbon group as A may be preferably a linear, branched, or cyclic alkyl group or aryl group, or more preferably a linear alkyl group (primary alcohol). Use of a primary alcohol may lead to further improvement in the reactivity and an increase in the yield.

The molecular weight of the compound represented by A-OH is preferably 32 or more, and preferably 500 or less, more preferably 300 or less, still more preferably 200 or less, or further preferably 100 or less.

Examples of the compound represented by A-OH include methanol, ethanol, isopropanol, isobutanol, n-propanol, n-butanol and the compounds shown below, among which methanol is preferred.

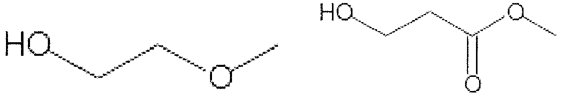

The compound represented by A-OH in the present embodiment may be the same substance as the solvent described in detail below. To illustrate this, both the compound represented by A-OH and the solvent may be methanol, for example.

In the oxidation-reduction reaction, the amount of the compound represented by A-OH (nucleophilic agent) relative to 1 mol of the substrate may be preferably 1 mol or more, more preferably more than 1 mol, further preferably 1.5 mol or more, and still further preferably 2.5 mol or more. Moreover, the amount of the compound represented by A-OH (nucleophilic agent) relative to 1 mol of the substrate may be preferably 20 mol or less, more preferably 10 mol or less, further preferably 8 mol or less, still further preferably 5 mol or less, or especially further preferably 4 mol or less.

The oxidation-reduction reaction according to the present embodiment may be performed with one type of the compound represented by A-OH alone or with two or more types of the compounds represented by A-OH. When two or more types are used, the total amount is preferably within the range described above.

When the compound represented by A-OH is also used as a solvent, the amount thereof used is similar to the preferred ranges of the amount of the solvent used.

In the production method of the present embodiment, an oxidation-reduction reaction is performed in the presence of a catalyst and a base. The catalyst is an N-heterocyclic carbene having a pKa of 33.0 or less, the pKa being calculated from the free energy of the stable structure of the catalytic molecule in methanol determined by solvation model density (SMD) using Gaussian 16 (hereinafter the pKa may be sometimes referred to simply as "catalytic pKa").

More specifically, the pKa of the catalyst was determined by structure optimization calculation of a gas state molecule under the condition of B3LYP/6-31 + G(d) using software Gaussian 16. Vibration modes were calculated for the obtained structure, and it was confirmed that the structure was a stable structure with no imaginary vibration. In calculation to determine a stable structure, the identical conformation was assumed for the conformations before and after acid dissociation except for the presence or absence of hydrogen. The free energies of molecules in the gas state and in solvent at 298.15K were determined by one-point calculation using M06-2X/6-311++G(d, p). Here, the solvation model was SMD in order to take the solvent effect into consideration. The solvent was methanol. The free energy of dissolution of proton was -255.6 kcal/mol. From the free energies of these states determined by this method, the free energy difference ΔGsoln before and after the acid-dissociation was calculated to determine the pKa.

By using a catalyst having a pKa of 33.0 or less as determined by the above method, the conversion rate of the alkylfuranaldehyde can be increased and the reaction rate (reaction cycle) of the reaction system can be increased.

The pKa of the catalyst may be preferably 32.0 or less, more preferably 31.5 or less, and further preferably 31.0 or less, 30.5 or less, 30.0 or less, 29.5 or less, 29.0 or less, 28.5 or less, 28.0 or less, 27.5 or less, 27.0 or less, 26.5 or less, 26.0 or less, 25.5 or less, and 25.0 or less, with an increase in preference in this order . When the pKa is not more than the upper limit as mentioned above, the yield of the alkylfurancarboxylic acid ester is further improved. The pKa of the catalyst is preferably 15.0 or more, more preferably 16.0 or more, and further preferably 17.0 or more, 17.5 or more, 18.0 or more, 18.5 or more, 19.0 or more, 19.5 or more, 20.0 or more, 20.5 or more, 21.0 or more, 21.5 or more, 22.0 or more, 22.5 or more, and 23.0 or more, with an increase in preference in this order.

The oxidation-reduction reaction in the present embodiment may use one type of the catalyst alone or may use two or more types of the catalysts. When two or more catalysts are used, each catalyst satisfies the preferable range above.

The catalyst having a pKa of 33.0 or less facilitates the reaction by causing the base to act.

The catalyst having a pKa of 33.0 or less preferably includes at least one type selected from the group consisting of triazolium, imidazolinium, imidazolium, and thiazolium, more preferably includes at least one type selected from the group consisting of triazolium, imidazolinium, and imidazolium, further preferably includes at least one type selected from the group consisting of triazolium and imidazolinium, and still further preferably includes at least one type selected from triazolium.

More specifically, the catalyst having a pKa of 33.0 or less preferably includes at least one type of catalyst represented by the following formula: 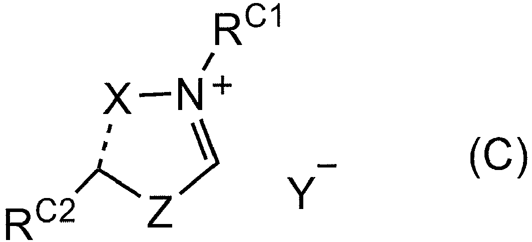 where each of R^{C1} and R^{C2} independently represents a hydrogen atom, an alkyl group having 1 to 12 carbon atom(s), an alkoxy group having 1 to 12 carbon atom(s), an aryl group having 6 to 18 carbon atoms, a heteroaryl group having 4 to 16 carbon atoms, a halogenated alkyl group having 1 to 12 carbon atom(s), a halogenated aryl group having 6 to 18 carbon atoms, an alkylaryl group having 7 to 20 carbon atoms, or an arylalkyl group having 7 to 20 carbon atoms; Z represents -S- or -NR^{c3}-; X is a methine group (=CR^{C5}-), a nitrogen atom (=N-) or a methylene group (-CR^{C6}₂-); in a case where X is a methine group or a nitrogen atom, the broken line in Formula (C) represents a double bond, and in a case where X is a methylene group, the broken line in Formula (C) represents a single bond; where each of R^{C3}, R^{C5}, and R^{C6} independently represents a hydrogen atom, an alkyl group having 1 to 12 carbon atom(s), an alkenyl group having 2 to 12 carbon atoms, an alkynyl group having 2 to 12 carbon atoms, an aryl group having 6 to 18 carbon atoms, an alkoxy group having 1 to 12 carbon atom(s), an acyl group having 2 to 12 carbon atoms, a hydroxy group, a carboxy group, or a halogen atom; Y⁻ represents a counter anion; and each pair of R^{C2} and R^{C3}, R^{C2} and R^{C5}, R^{C2} and R^{C6}, R^{C1} and R^{C5}, and R^{C1} and R^{C6} may be independently bonded or condensed with each other to form a ring.

Two R^{C6} s included in the methylene group (-CR^{C6}₂-) may be the same as or different from each other. Hereinafter, when two or more groups having the same symbol are present in one compound, the two or more groups having the same symbol may be the same as or different from each other.

In the present embodiment, the term "alkyl group" may refer to a cycloalkyl group in addition to a linear or branched alkyl group. The terms "alkylaryl group" and "arylalkyl group" may additionally refer to a group in which a cyclic alkyl and an aryl (for example, a benzene ring) are condensed together.

R^{C1} and R^{C2} each independently represent a hydrogen atom, an alkyl group having 1 to 6 carbon atom(s), an alkoxy group having 1 to 12 carbon atom(s), an aryl group having 6 to 12 carbon atoms, a halogenated alkyl group having 1 to 6 carbon atom(s), a halogenated aryl group having 6 to 12 carbon atoms, an alkylaryl group having 7 to 13 carbon atoms, or an arylalkyl group having 7 to 13 carbon atoms. Each halogen atom in the halogenated alkyl group or the halogenated aryl group may be preferably a fluorine atom or a chlorine atom, independently.

It is preferable that R^{C3}, R^{C5}, and R^{C6} be each independently a hydrogen atom, an alkyl group having 1 to 6 carbon atom(s), an alkenyl group having 2 to 6 carbon atoms, an alkynyl group having 2 to 6 carbon atoms, an aryl group having 6 to 12 carbon atoms, an alkoxy group having 1 to 6 carbon atom(s), an acyl group having 2 to 7 carbon atoms, a hydroxy group, a carboxy group, or a halogen atom. It is more preferable that R^{C3}, R^{C5}, and R^{C6} be each independently a hydrogen atom, an alkyl group having 1 to 6 carbon atom(s), or an aryl group having 6 to 12 carbon atoms. It is further more preferable that R^{C3}, R^{C5}, and R^{C6} be each independently a hydrogen atom, or an alkyl group having 1 to 3 carbon atom(s) . It is still further preferable that R^{C3}, R^{C5}, and R^{C6} be each a hydrogen atom.

Y⁻ is any monovalent anion, but may be preferably an anion containing a halogen, or more preferably F⁻, Cl⁻, Br⁻, I⁻, or BF₄⁻.

In a case where the catalyst represented by Formula (C) is such that X is a methine group, the catalyst is preferably represented by the following formula (C2): 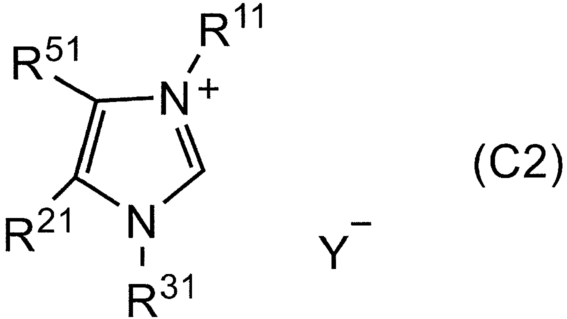 where, in Formula (C2), R¹¹ represents a group as defined for the R^{C1}, R²¹ represents a group as defined for the R^{C2}, R³¹ represents a group as defined for the R^{C3}, R⁵¹ represents a group as defined for the R^{C5}, Y⁻ represents a counter anion, and each pair of R²¹ and R³¹, R²¹ and R⁵¹, and R¹¹ and R⁵¹ may be independently bonded or condensed with each other to form a ring.

It is preferable that R¹¹ and R³¹ each be independently an alkyl group having from 1 to 12 carbon atom(s), and it is more preferable that R¹¹ and R³¹ each be independently an alkyl groups having from 1 to 6 carbon atom(s).

R²¹ and R⁵¹ are each preferably a hydrogen atom.

In a case where the catalyst represented by Formula (C) is such that X is a methylene group, it is preferably represented by the following formula (C3): 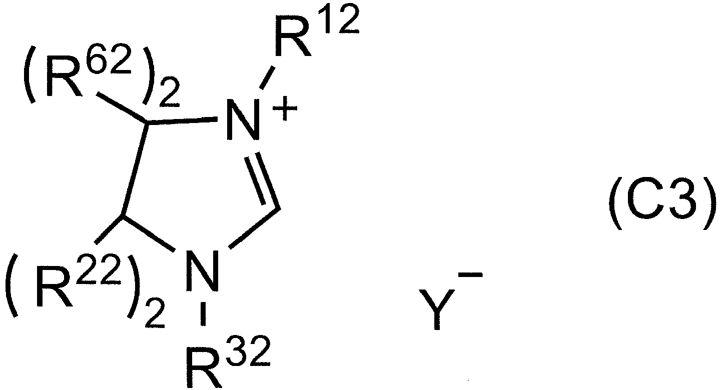 where, in Formula (C3), R¹² represents a group as defined for the R^{C1}, R²² represents a group as defined for the R^{C2}, R³² represents a group as defined for the R^{C3}, R⁶² represents a group as defined for the R^{C6}, Y⁻ represents a counter anion, and each pair of R²² and R³², R²² and R⁶², and R¹² and R⁶² may be independently bonded or condensed with each other to form a ring.

It is preferable that R¹² and R³² each be independently an alkyl group having from 1 to 12 carbon atom(s), an aryl group having from 6 to 18 carbon atoms, or an arylalkyl group having from 7 to 20 carbon atoms.

R²² and R⁶² are each preferably a hydrogen atom.

When the catalyst represented by Formula (C) is such that X is a nitrogen atom, the catalyst is preferably represented by the following formula (C4): 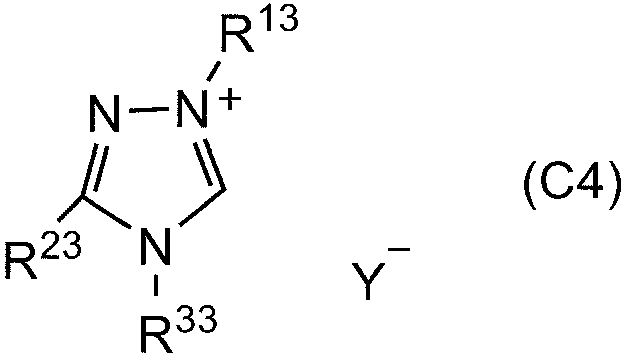 where, in Formula (C4), R¹³ represents a group as defined for the R^{C1}, R²³ represents a group as defined for the R^{C2}, R³³ represents a group as defined for the R^{C3}, Y⁻ represents a counter anion, and R²³ and R³³ may be bonded or condensed to each other to form a ring.

It is preferable that each of R¹³ and R³³ be preferably an alkyl group having 1 to 12 carbon atom(s), it is more preferable that each of R¹³ and R³³ be an alkyl group having from 1 to 5 carbon atom(s), it is still more preferable that each of R¹³ and R³³ be an alkyl group having 1 to 3 carbon atom(s), and it is particularly preferable that each of R¹³ and R³³ be a methyl group.

R²³ is preferably a hydrogen atom.

In a case where the catalyst represented by Formula (C4) is such that R²³ and R³³ form a ring, the catalyst is preferably represented by the following formula (C5): 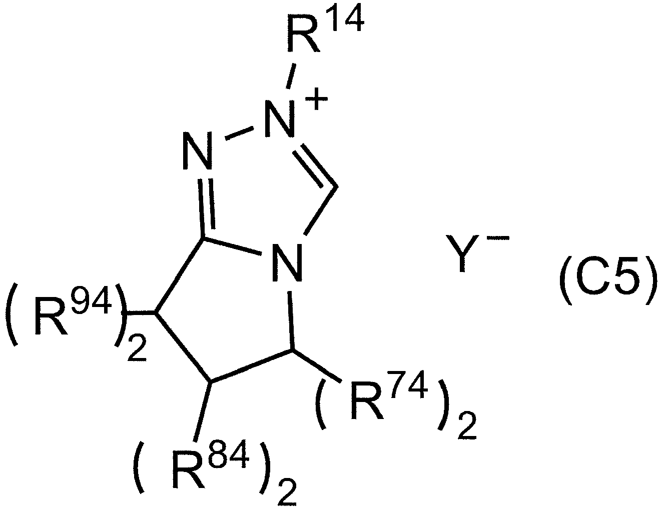 where, in Formula (C5), R¹⁴ represents a group as defined for the R^{C1}, R⁷⁴, R⁸⁴, and R⁹⁴ each independently represent a group as defined for the R^{C2}, Y⁻ represents a counter anion.

R¹⁴ is preferably a halogenated aryl group having 6 to 18 carbon atoms or an alkylaryl group having 7 to 20 carbon atoms.

R⁷⁴, R⁸⁴ and R⁹⁴ are preferably a hydrogen atom.

In a case where the catalyst represented by Formula (C) is such that Z is a sulfur atom, the catalyst is preferably represented by the following formula (C4): 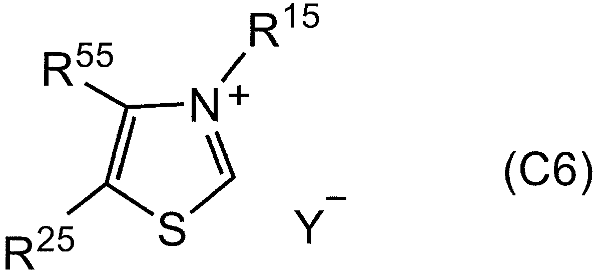 where, in Formula (C6), R¹⁵ represents a group as defined for the R^{C1}, R²⁵ represents a group as defined for the R^{C2}, R⁵⁵ represents a group as defined for the R^{C5}, Y⁻ represents a counter anion, and each pair of R¹⁵ and R⁵⁵, and R⁵⁵ and R²⁵ may be independently bonded or condensed to each other to form a ring.

In the present embodiment, it is preferable that the catalyst is one represented by formula (C4).

Examples of the catalyst include the following compounds. Needless to say, the present invention is not limited to these.

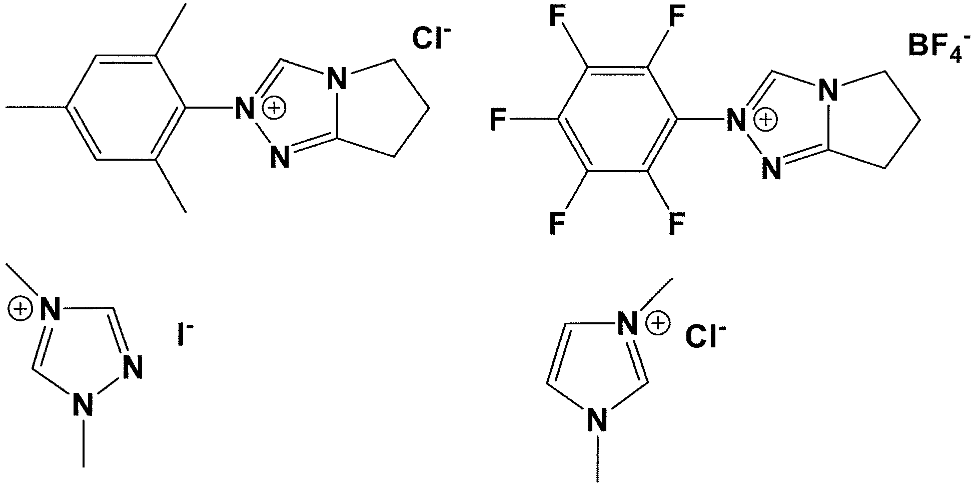

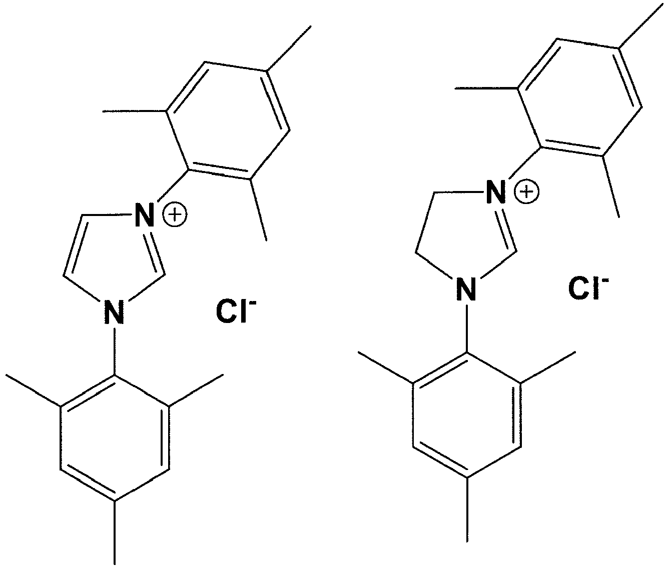

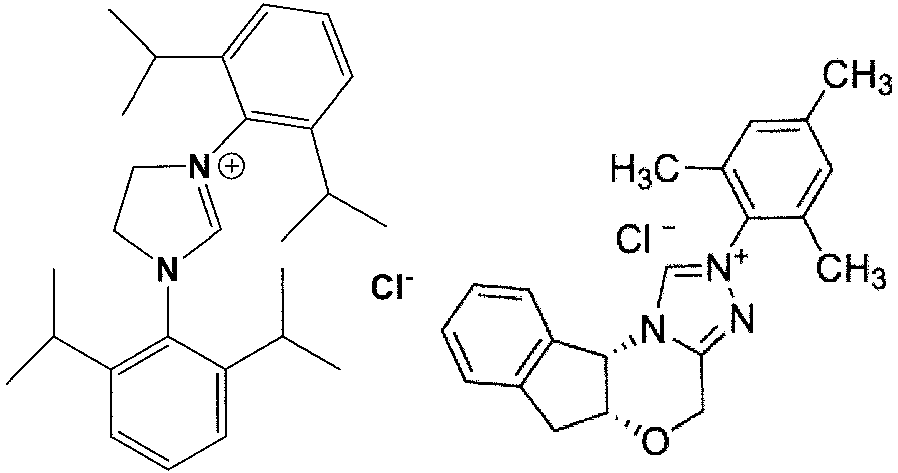

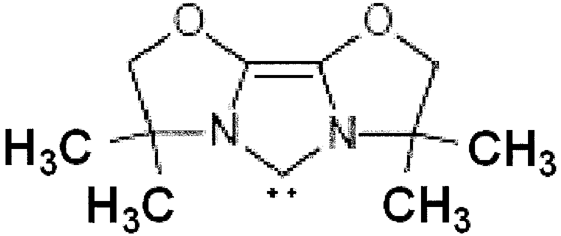

In the oxidation-reduction reaction, the amount of the catalyst having a pKa of 33.0 or less relative to 1 mol of the substrate is preferably 0.0001 mol or more, more preferably 0.001 mol or more, further preferably 0.01 mol or more, still further preferably 0.04 mol or more, still further preferably 0.05 mol or more, still more further preferably 0.1 mol or more, or especially more preferably 0.2 mol or more. The amount of the catalyst not less than the lower limit facilitates more efficient progress of the oxidation-reduction reaction. In addition, the amount of the catalyst relative to 1 mol of the substrate is preferably 5.0 mol or less, more preferably 3.0 mol or less, still more preferably 1.0 mol or less, further preferably 0.5 mol or less, or still further preferably 0.4 mol or less.

In the oxidation-reduction reaction of the present embodiment, the catalyst having a pKa of 33.0 or less used therein may include one type thereof alone, or may include two or more types thereof. When two or more types are used, the total amount is preferably within the range described above.

In the production method of the present embodiment, it is more preferable that the oxidation-reduction reaction be performed in the presence of an oxidizing agent. The use of the oxidizing agent can suppress the side reaction more effectively.

The type of the oxidizing agent is not particularly limited and may be an organic compound or an inorganic compound.

The amount of the oxidizing agent can be appropriately determined depending on the kind of the oxidizing agent, and for example, the amount of the oxidizing agent is preferably 0.01 mol or more, and preferably 20.0 mol or less with respect to 1 mol of the substrate.

In the oxidation-reduction reaction of the present embodiment, the oxidizing agent used therein may include one type thereof alone, or may include two or more types thereof. When two or more types are used, the total amount is preferably within the range described above.

In the present embodiment, when the oxidizing agent is an organic compound, examples thereof include; hypervalent organic iodine compounds such as quinone, nitrobenzene, and 2-iodobenzoic acid; nitroxyl radical compounds such as 2,2,6,6-tetramethylpiperidine-1-oxyl (TEMPO); and organic peroxides such as m-chlorobenzoic acid, and it is preferable that the oxidizing agent be quinone. Here, the quinone refers to a compound in which two hydrogen atoms bonded to a benzene ring of an aromatic hydrocarbon are each substituted with an oxygen atom.

In addition, oxidizing agents described in "Oxidizing Agents for Organic Synthesis, Third Edition" published by Fujifilm Wako Pure Chemical Industries, Ltd. can also be used, and the contents thereof are incorporated into the present specification.

The molecular weight of quinone as the oxidizing agent may be preferably 108 or more, and preferably 1000 or less, more preferably 800 or less, or acceptably 600 or less.

It is preferable that the oxidizing agent include at least one oxidizing agent represented by formula (O): 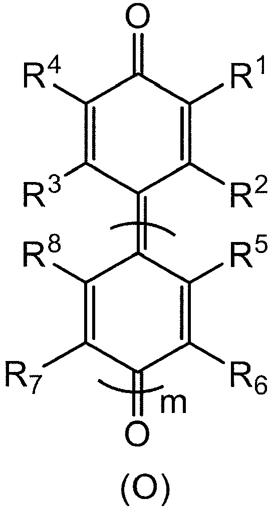 where each of R¹ to R⁸ independently represents a hydrogen atom, an alkyl group having 1 to 12 carbon atom(s), an alkenyl group having 2 to 12 carbon atoms, an alkynyl group having 2 to 12 carbon atoms, an alkoxy group having 1 to 12 carbon atom(s), an aryl group having 6 to 18 carbon atoms, an acyl group having 2 to 12 carbon atoms, or a hydroxy group; in a case where R¹ to R⁸ each are independently such an alkyl group, an alkenyl group, an alkynyl group, an alkoxy group, an aryl group, or an acyl group, the groups of R¹ to R⁸ are each independently unsubstituted or substituted with a substituent bonded to a carbon atom in the group, the substituent being an alkyl group having 1 to 6 carbon atom(s), an alkoxy group having 1 to 6 carbon atom(s), a hydroxy group, a halogen atom, a cyano group, or a nitro group; m is 1 or 0; and each pair of R¹ and R², R³ and R⁴, R⁵ and R⁶, and R⁷ and R⁸ may be independently bonded or condensed to each other to form a ring.

It is preferable that R¹ to R⁸ each be independently a hydrogen atom, an alkyl group having 1 to 12 carbon atom(s), or an alkoxy group having 1 to 12 carbon atom(s). It is more preferable that R¹ to R⁸ each be independently a hydrogen atom, an alkyl group having 1 to 6 carbon atom(s), or an alkoxy group having 1 to 8 carbon atom(s). It is still more preferable that R¹ to R⁸ each be independently a hydrogen atom or an alkyl group having 1 to 6 carbon atom(s).

An example in which m in Formula (O) is 0 and each pair of R¹ and R², and R³ and R⁴ is condensed with each other to form a 6-membered ring is the following Formula (O-1): 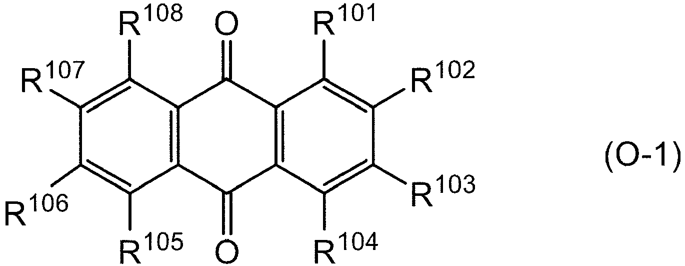 wherein R¹⁰¹ to R¹⁰⁸ in Formula (O-1) each independently represent a group as defined for the R¹ in Formula (O).

R¹⁰¹ to R¹⁰⁸ each independently represent a group as defined for the R¹, and, preferably, may be independently a hydrogen atom or an alkyl group containing from 1 to 6 carbon atom(s).

An example in which m in Formula (O) is 1 and each pair of R¹ and R², R³ and R⁴, R⁵ and R⁶, and R⁷ and R⁸ is condensed with each other to form a 6-membered ring is the following Formula (O-2): 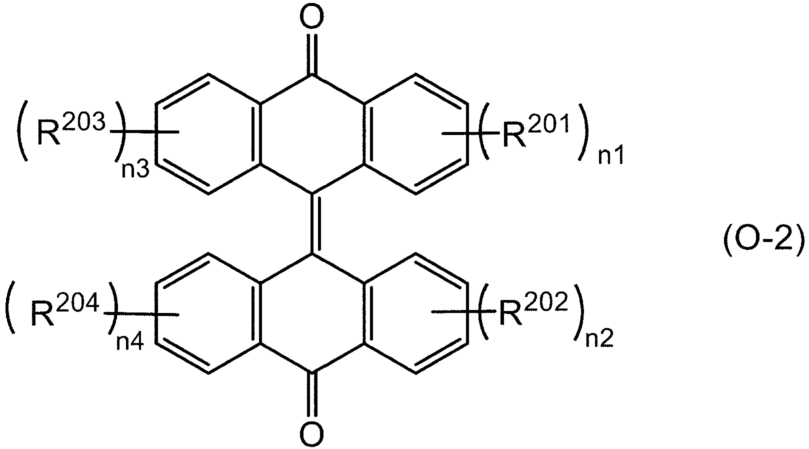 where, in Formula (O-2), R²⁰¹ to R²⁰⁴ each independently represent a group as defined for the R¹ in Formula (O).

R²⁰¹ to R²⁰⁴ each independently represent a group as defined for the R¹, and is preferably a hydrogen atom. n1, n2, n3 and n4 each independently represent an integer of 1 to 4.

An example in which m in Formula (O) is 1 and each pair of R¹ and R², R³ and R⁴, R⁵ and R⁶, or R⁷ and R⁸ is not condensed with each other to form a ring is the following Formula (O-3): 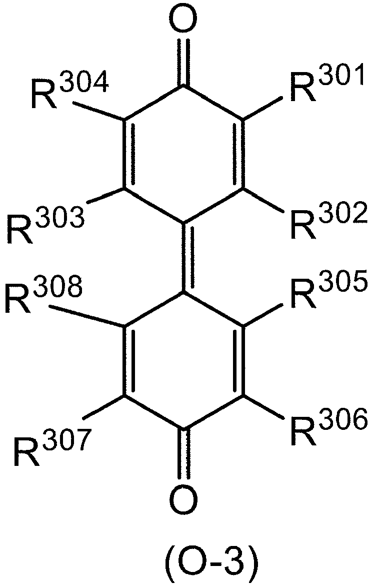 where each of R³⁰¹ to R³⁰⁸ in Formula (O-3) independently represents a group as defined for the R¹ in Formula (O).

R³⁰¹ to R³⁰⁸ each independently represent a group as defined for the R¹, and, R³⁰¹ to R³⁰⁸ each independently are preferably a hydrogen atom or an alkyl group having 1 to 12 carbon atom(s), or more preferably a hydrogen atom or an alkyl group having 1 to 8 carbon atom(s).

Examples in which m in Formula (O) is 0 and each pair of R¹ and R², and R³ and R⁴ is not condensed with each other to form a ring include the following Formula (O-4): 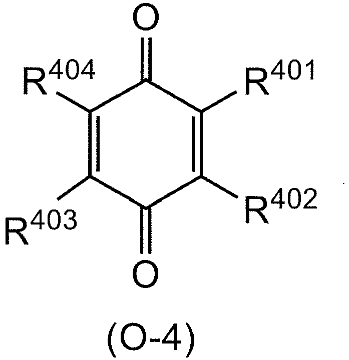 where R⁴⁰¹ to R⁴⁰⁴ in Formula (O-4) each independently represent a group as defined for the R¹ in Formula (O).

R⁴⁰¹ to R⁴⁰⁴ each independently represent a group as defined for the R¹ above, and, R⁴⁰¹ to R⁴⁰⁴ each independently are preferably a hydrogen atom, an alkyl group having 1 to 12 carbon atom(s), or an alkoxy group having 1 to 12 carbon atom(s), more preferably a hydrogen atom or an alkyl group having 1 to 12 carbon atom(s), or still more preferably a hydrogen atom or an alkyl group having 1 to 8 carbon atom(s). In a case where R⁴⁰¹ to R⁴⁰⁴ are independently an alkyl group or an alkoxy group, the alkyl group or the alkoxy group may have a halogen atom as a substituent bonded to a carbon atom in the group.

In the present embodiment, Formula (O-1), Formula (O-3), and Formula (O-4) are preferable, Formula (O-3) and Formula (O-4) are more preferable, and Formula (O-3) is still more preferable.

Examples of the oxidizing agent include the following compounds. Needless to say, the present invention is not limited to these. Note that tBu is a tert-butyl group.

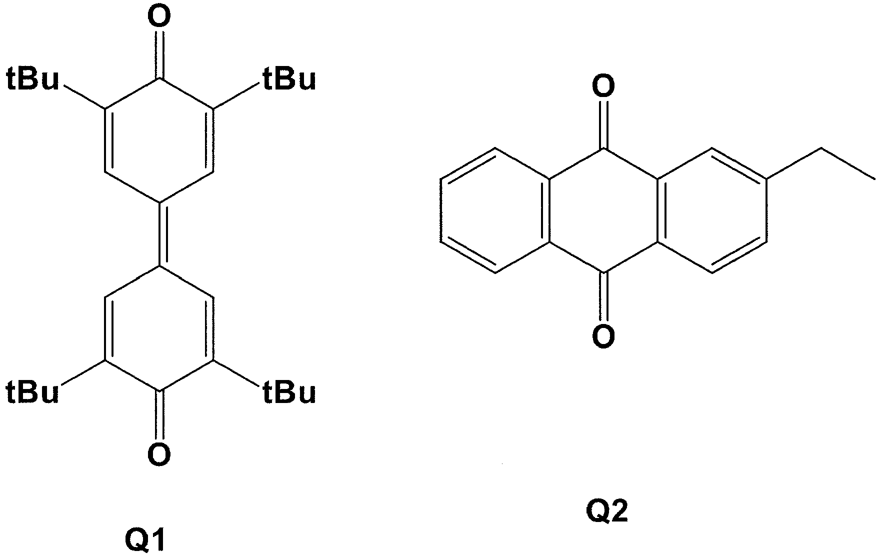

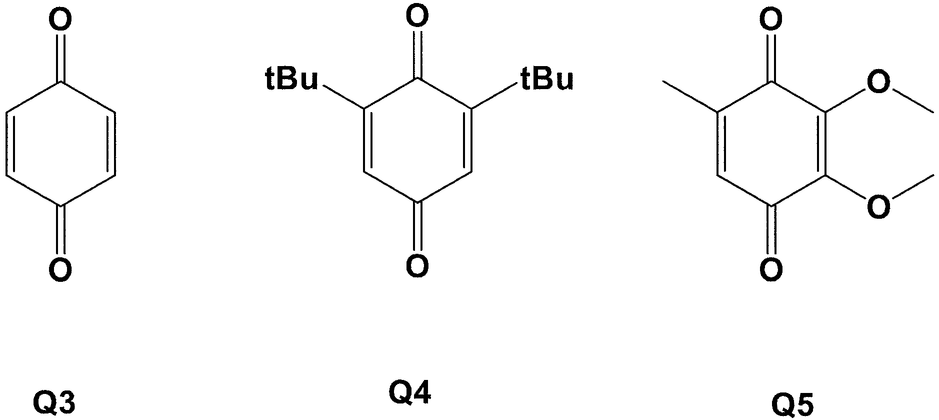

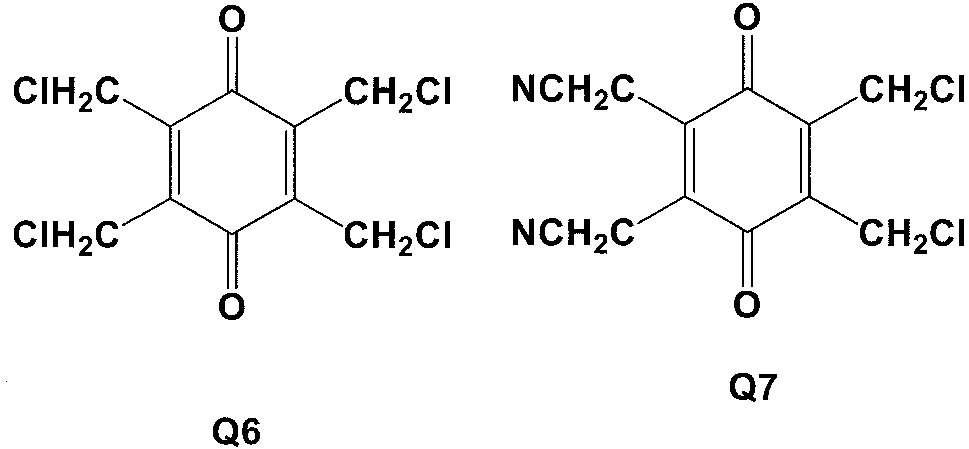

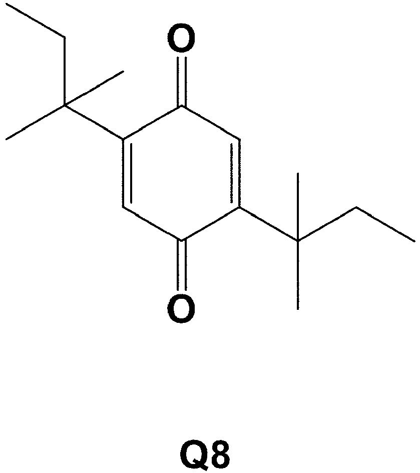

In the oxidation-reduction reaction, the amount of the quinone relative to 1 mol of the substrate is preferably 0.01 mol or more, more preferably 0.05 mol or more, further preferably 0.1 mol or more, still further preferably 0.5 mol or more, or still further preferably 0.8 mol or more. The amount of the quinone equal to or more than the lower limit facilitates a further improvement of the yield of the alkylfurancarboxylic acid ester. The amount of the oxidizing agent with respect to 1 mol of the substrate may be 3.0 mol or less, 2.5 mol or less, 2.0 mol or less, 1.5 mol or less, or 1.2 mol or less.

In the oxidation-reduction reaction, the quinone may include one type of quinone alone, or may include two or more types of quinone. When two or more types are used, the total amount is preferably within the range described above.

In the present embodiment, in a case where the oxidizing agent is an inorganic compound, examples thereof include permanganates such as potassium permanganate; chromic acids such as potassium dichromate and chromium oxide; nitric acids such as nitric acid and potassium nitrate; halogens such as fluorine, chlorine, bromine, and iodine; peroxides such as aqueous hydrogen peroxide, and sodium peroxide; oxides such as copper (II) oxide, lead (IV) oxide, and manganese (IV) oxide; and metal salts such as iron chloride, and copper sulfate. The oxidizing agent may be preferably oxides, or more preferably manganese oxide.

In the oxidation-reduction reaction, the amount of the inorganic compound as the oxidizing agent relative to 1 mol of the substrate is preferably 1.00 mol or more, more preferably 3.00 mol or more, and still more preferably 5.00 mol or more, and is preferably 20.0 mol or less, more preferably 15.0 mol or less, and still more preferably 10.0 mol or less.

In the oxidation-reduction reaction of the present embodiment, the inorganic compound as the oxidizing agent used therein may include one type thereof, or may include two or more types thereof. When two or more types are used, the total amount is preferably within the range described above.

In the production method of the present embodiment, the oxidation-reduction reaction is preferably performed in the presence of a solvent.

The solvent is not particularly limited as long as it can dissolve a part or all of the alkylfuranaldehyde and does not interfere with the oxidation-reduction reaction.

Examples of the solvent usable in the production method of the present embodiment include aromatic hydrocarbon solvents, aliphatic hydrocarbon solvents, amide solvents, ether solvents, alcohol solvents, halogen solvents, and ester solvents. Aromatic hydrocarbon solvents, ether solvents, or alcohol solvents are preferable, and ether solvents and sulfoxide solvents are more preferable.

Specific examples of the aromatic hydrocarbon solvents include benzene, and toluene.

Specific examples of the amide solvents include acetonitrile, N,N-dimethylacetamide, and N,N-dimethylformamide.

Specific examples of the ether solvents include tetrahydrofuran (hereinafter, also referred to as THF), and diethyl ether.

Specific examples of the alcohol solvents include methanol, ethanol, and isopropanol. The alcohol solvent can also act as a nucleophilic agent.

Specific examples of the halogen solvents include dichloromethane, dichloroethane, and chloroform.

Specific examples of the ester solvents include ethyl acetate.

Specific examples of the sulfoxide solvent include dimethyl sulfoxide.

In the oxidation-reduction reaction, the amount of the solvent to be used is not particularly limited, but is preferably 0.5 times by mass or more, more preferably 0.8 times by mass or more, and still more preferably 1.0 times by mass or more relative to the substrate (alkylfuranaldehyde) from the viewpoint of productivity and energy efficiency. The amount of the solvent to be used may be preferably 200 times by mass or less, more preferably 100 times by mass or less, even more preferably 50 times by mass or less, still more preferably 40 times by mass or less, and still more preferably 30 times by mass or less, relative to the substrate.

In the oxidation-reduction reaction, the solvent may include one type thereof alone, or may include two or more types thereof. When two or more types are used, the total amount thereof is preferably within the above range.

In the production method of the present embodiment, the oxidation-reduction reaction is preferably performed in the presence of a base. When the reaction is carried out in the presence of a base, the improvement of the yield of the alkylfurancarboxylic acid ester is further facilitated.

The base may be an organic base or an inorganic base. Examples of the base include alkylamine, alkanolamine, polyamine, hydroxylamine, cyclic amine, quaternary ammonium, alkali metal-containing compounds and alkaline earth metal-containing compounds. Alkylamine, polyamine, cyclic amine and alkali metal-containing compounds are preferable, diazabicycloundecene, sodium carbonate, sodium hydroxide, triethylamine, sodium methoxide and potassium carbonate are more preferable. The base may preferably include at least one of diazabicycloundecene, sodium carbonate or potassium carbonate, or more preferably diazabicycloundecene and/or sodium carbonate, or further preferably diazabicycloundecene.

In the oxidation-reduction reaction, the amount of the base is preferably 1 mol or more relative to 1 mol of the catalyst. When the amount of the base is not less than the lower limit, the oxidation-reduction reaction proceeds more effectively. Furthermore, the amount of the base may be preferably 200 mol or less, or more preferably 100 mol or less, relative to 1 mol of the substrate. In the oxidation-reduction reaction of the present embodiment, the base may include one type thereof alone, may include two or more types thereof. When two or more types are used, the total amount is preferably within the range described above.

The reaction temperature of the oxidation-reduction reaction in the production method of the present embodiment is not particularly limited, but may be -80°C or higher, more preferably 0°C or higher, further preferably 15°C or higher, still further preferably 20°C or higher, or especially preferably 25°C or higher. The reaction temperature of the oxidation-reduction reaction in the production method may be preferably 200°C or lower, more preferably 150°C or lower, further preferably 100°C or lower, still further preferably 50°C or lower, and especially preferably 40°C or lower.

In the production method of the present embodiment, the reaction temperature may be the constant except for the initial temperature increase and the final temperature decrease (provided that variations of ±5°C are allowed), and the reaction temperature may be varied in two or more stages. In the present embodiment, it is preferable that the reaction temperature is constant (provided that provided that variations of ±5°C are allowed) except for the initial temperature increase and the final temperature decrease.

The reaction time of the oxidation-reduction reaction in the production method of the present embodiment is preferably 1 min or longer, may be 30 min or longer, may be 1 hour or longer, and may be 1.5 hours or longer. The reaction time of the oxidation-reduction reaction is preferably 50 hours or less, may be 30 hours or less, and may be 25 hours or less.

The present embodiment describes an illustrative example of the production of an alkylfurancarboxylic acid ester, in which a solvent, a catalyst, a base, and a compound represented by A-OH are added to an alkylfuranaldehyde as a substrate in this order.

The separation of the reaction mixture from the catalyst after the reaction can be performed by a typical method, such as precipitation, centrifugation, or filtration. Depending on the catalyst used, to prevent ignition, the separation of the catalyst is preferably performed in an inert gas atmosphere, such as nitrogen or argon, as appropriate. Furthermore, the reaction mixture is such that, after the resultant reaction solution is condensed if needed, the residue as such may be directly used as a raw material or an intermediate. As an alternative, the reaction mixture may be subjected to an appropriate post-treatment for purification. Specific examples of the method for the post-treatment include well-known purification methods, such as extraction, distillation, and chromatography. A combination of two or more types of these purification methods may be performed.

In the production method of the present embodiment, the higher the conversion rate of the raw material, the better. The conversion rate is preferably 50 mol% or more, and more preferably 80 mol% or more. The upper limit is 100 mol%, ideally.

In the production method of the present embodiment, the higher the yield of the alkylfurancarboxylic acid ester, the better. The yield is preferably 3% or more, more preferably 10% or more, or further preferably 30% or more. The upper limit is 100 mol%, ideally.

In the production method of the present embodiment, usually, a salt is not produced as a by-product. Therefore, the obtained alkylfurancarboxylic acid ester is preferably used for various applications. In particular, the alkylfurancarboxylic acid ester obtained by the production method of the present embodiment is preferably used as various industrial materials or raw materials thereof.

### Examples

The present invention will be described more specifically with reference to examples below. Materials, amounts used, proportions, processing details, processing procedures, and the like described in the following examples can be appropriately changed as long as they do not depart from the spirit of the present invention. Thus, the scope of the present invention is not limited to the specific examples described below.

If a measuring device used in the examples is not readily available due to discontinuation or the like, another device with equivalent performance can be used for measurement.

### Raw material

### Substrates

MFF: methylfurfural, available from Fujifilm Wako Pure Chemical Industries, Ltd., 133-11771
IPrFF: isopropylfurfural, available from Matrix Scientific, 020867

### Solvents

THF: tetrahydrofuran, available from Fujifilm Wako Pure Chemical Industries, Ltd., 206-00483
Toluene: available from FUJIFILM Wako Pure Chemical Industries, Ltd., 204-01866
Methanol: available from Fujifilm Wako Pure Chemical Industries, Ltd., 137-01823
Dichloromethane : available from Fujifilm Wako Pure Chemical Industries, Ltd., 135-02441

### <Base>

DBU: diazabicycloundecene, available from Tokyo Chemical Industry Co., Ltd., D1270
Sodium carbonate: available from Fujifilm Wako Pure Chemical Industries, Ltd., 199-01585

### Nucleophilic agents

MeOH (methanol): available from Fujifilm Wako Pure Chemical Industries, Ltd., 137-01823
NPrOH (n-propyl alcohol): available from Tokyo Chemical Industry Co., Ltd., P0491

### <Catalyst>

CatA : available from Tokyo Chemical Industry Co., Ltd., D4624 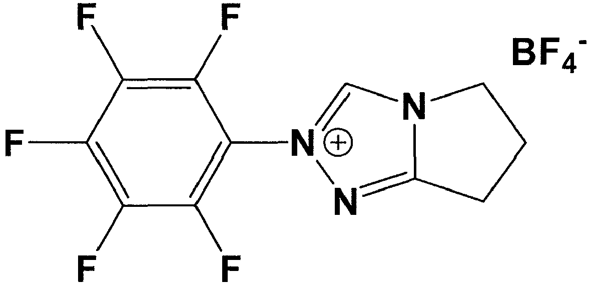
CatB : available from Sigma-Aldrich, 688487-250MG 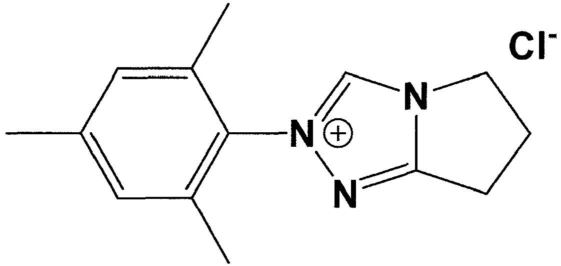
CatC : available from Tokyo Chemical Industry Co., Ltd., D3341 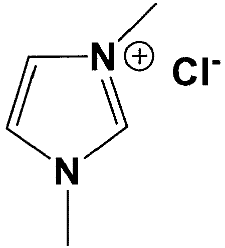
CatD : available from Tokyo Chemical Industry Co., Ltd., D3711 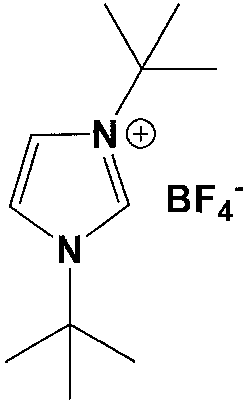
CatE : available from Sigma-Aldrich, 708607-1G 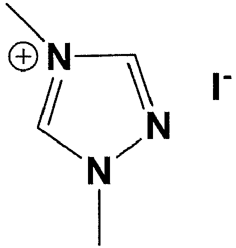
CatF : available from Tokyo Chemical Industry Co., Ltd., D3446 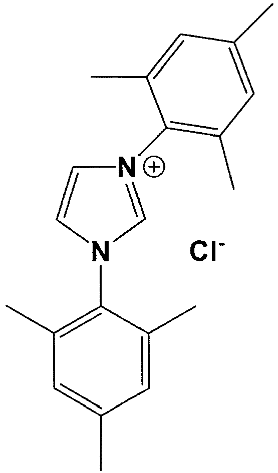
CatG : available from Tokyo Chemical Industry Co., Ltd., B3158

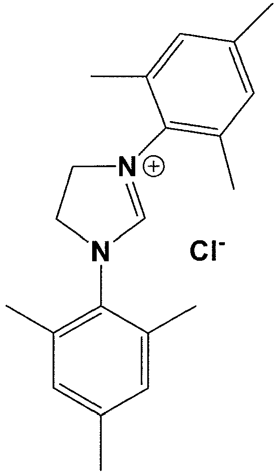

### <Oxidizing agents>

Q1 : available from FUJIFILM Wako Pure Chemical Industries, Ltd., 359-16793 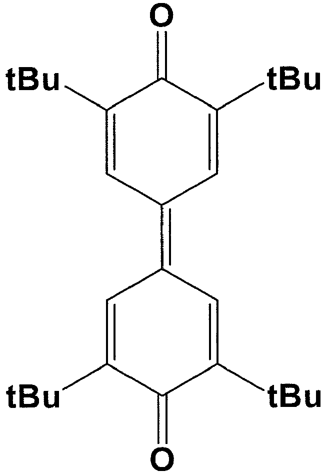
tBu is a tert-butyl group.
Q2 : available from Tokyo Chemical Industry Co., Ltd., E0063 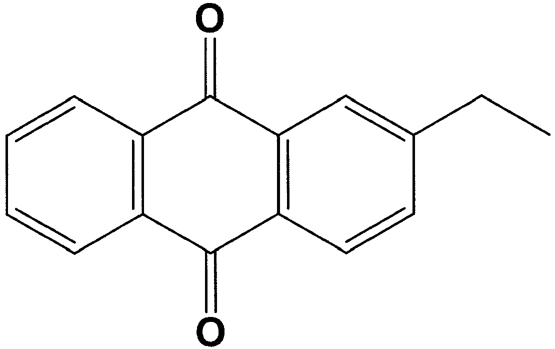
Q3 : available from FUJIFILM Wako Pure Chemical Industries, Ltd., 171-00242 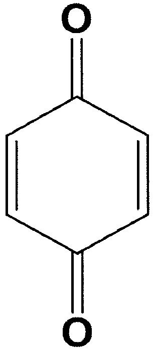
Q4 : available from Tokyo Chemical Industry Co., Ltd., D2256 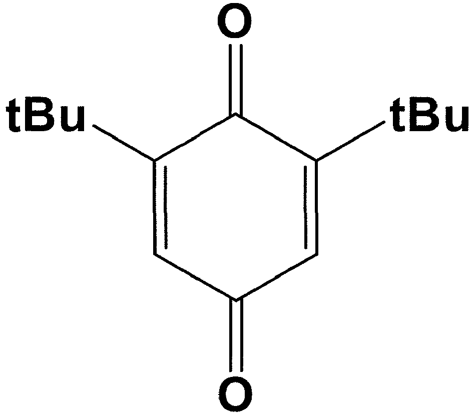
Q8 : available from Tokyo Chemical Industry Co., Ltd., D2152 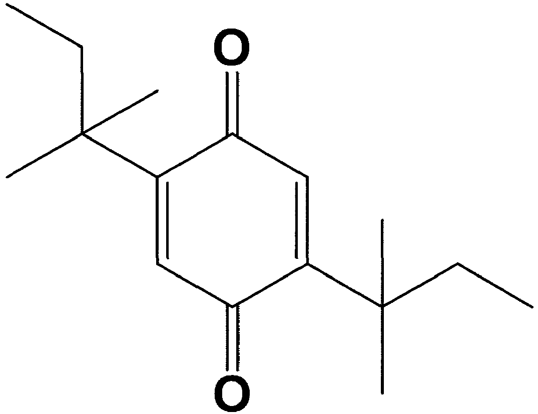
Q9 : manganese (IV) oxide : available from Fujifilm Wako Pure Chemical Industries, Ltd., 138-09675

### Example 1

### <Production of methyl furoate>

In to a 20-mL eggplant flask, 0.033 g (0.3 mmol) of MFF, 1 g of THF (without a stabilizer), 0.033 g (0.3 eq, 0.09 mmol) of CatA, 0.014 g (0.3 eq) of DBU, and 0.29 g (3 eq, 0.9 mmol) of methanol were charged in this order. The flask was placed in a dry bath and stirred at 30°C for 2 h. The reaction solution after the reaction was analyzed using high performance liquid chromatography (HPLC) and quantified by an absolute calibration curve method.

The analytical conditions of the HPLC were as follows.
Apparatus : available from Shimadzu Corporation
Column : GL Science ODS-4 (250 mm × 46 mm, film thickness 5µm)
Mobile phases : Water (A) 70% : Acetonitrile (B) 30% → (5 min) → (A) 70% : (B) 30 0→(30 min) → (A) 0% : (B) 100% -> (10 min) → (A) 0% : (B) 100%
Column temperature: 40°C
Flow rate: 0.5 mL/min
Detector: UV (254 nm)

### Examples 2 to 18 and Comparative Examples 1 and 2

The production and analysis were conducted as in Example 1, except that some changes were made as shown in Table 1 or Table 2 below. The results are shown in Table 1 and Table 2.

In the case of the examples in which an oxidizing agent was added, the oxidizing agent was added in the amount shown in Table 1 or Table 2, after the catalyst.

The yields in Examples 17 and 18 are values after stirring for 3 hours, and the values in parentheses are values after stirring for 24 hours.

**[Table 1]**

| | Example 1 | Example 2 | Example 3 | Comparative Example 1 | Example 4 | Example 5 |
|---|---|---|---|---|---|---|
| Substrate | MFF | MFF | MFF | MFF | MFF | MFF |
| Solvents | THF | THF | THF | THF | THF | THF |
| Bases | DBU | DBU | DBU | DBU | DBU | DBU |
| Nucleophilic Agents | MeOH | MeOH | MeOH | MeOH | MeOH | MeOH |
| Catalysts | CatA | CatB | CatC | CatD | CatE | CatF |
| Oxidizing Agents | None | None | None | None | Q1 | Q1 |
| Substrates (mmol) | 0.3 | 0.3 | 0.3 | 0.3 | 0.3 | 0.3 |
| Solvents (g) | 1 | 1 | 1 | 1 | 1 | 1 |
| Bases (mmol) | 0.09 | 0.09 | 0.09 | 0.09 | 0.09 | 0.09 |
| Catalysts (mmol) | 0.09 | 0.09 | 0.09 | 0.09 | 0.09 | 0.09 |
| Nucleophilic agents (mmol) | 0.9 | 0.9 | 0.9 | 0.9 | 0.9 | 0.9 |
| Oxidizing agents (mmol) | None | None | None | None | 0.3 | 0.3 |
| Reaction temperature (°C) | 30 | 30 | 30 | 30 | 30 | 30 |
| Reaction time (hr) | 2 | 2 | 2 | 2 | 2 | 2 |
| pKa (calculated for pKa in MeOH) | 19.2 | 23.5 | 29.7 | 34.0 | 24.1 | 27.3 |
| Yield | 13% | 8% | 4% | 0% | 98% | 97% |
| Conversion rate | 100% | 100% | 100% | 5% | 100% | 100% |

| | Example 6 | Example 7 | Example 8 | Example 9 | Example 10 |
|---|---|---|---|---|---|
| Substrate | MFF | iPrFF | MFF | MFF | MFF |
| Solvents | THF | THF | THF | THF | THF |
| Bases | DBU | DBU | DBU | DBU | DBU |
| Nucleophilic Agents | MeOH | MeOH | nPrOH | MeOH | MeOH |
| Catalysts | CatG | CatE | CatE | CatE | CatE |
| Oxidizing Agents | Q1 | Q1 | Q1 | Q2 | Q3 |
| Substrates (mmol) | 0.3 | 0.3 | 0.3 | 0.3 | 0.3 |
| Solvents (g) | 1 | 1 | 1 | 1 | 1 |
| Bases (mmol) | 0.09 | 0.09 | 0.09 | 0.09 | 0.09 |
| Catalysts (mmol) | 0.09 | 0.09 | 0.09 | 0.09 | 0.09 |
| Nucleophilic agents (mmol) | 0.9 | 0.9 | 0.9 | 0.9 | 0.9 |
| Oxidizing agents (mmol) | 0.3 | 0.3 | 0.3 | 0.3 | 0.3 |
| Reaction temperature (°C) | 30 | 30 | 30 | 30 | 30 |
| Reaction time (hr) | 2 | 2 | 2 | 2 | 2 |
| pKa (calculated for pKa in MeOH) | 28.5 | 24.1 | 24.1 | 24.1 | 24.1 |
| Yield | 67% | 93% | 95% | 49% | 59% |
| Conversion rate | 100% | 99% | 100% | 100% | 88% |

**[Table 2]**

| | Example 11 | Example 12 | Comparative Example 2 | Example 13 | Example 14 |
|---|---|---|---|---|---|
| Substrate | MFF | MFF | MFF | MFF | MFF |
| Solvents | THF | THF | THF | Toluene | Methanol |
| Bases | DBU | DBU | DBU | Sodium carbonate | Sodium carbonate |
| Nucleophilic Agents | MeOH | MeOH | MeOH | MeOH | MeOH |
| Catalysts | CatE | CatE | CatD | CatE | CatE |
| Oxidizing Agents | Q4 | Q8 | Q1 | None | None |
| Substrates (mmol) | 0.3 | 0.3 | 0.3 | 1 | 1 |
| Solvents (g) | 1 | 1 | 1 | 1 | 1 |
| Bases (mmol) | 0.09 | 0.09 | 0.09 | 0.3 | 0.3 |
| Catalysts (mmol) | 0.09 | 0.09 | 0.09 | 0.3 | 0.3 |
| Nucleophilic agents (mmol) | 0.9 | 0.9 | 0.9 | 3 | 41 |
| Oxidizing agents (mmol) | 0.3 | 0.3 | 0.3 | None | None |
| Reaction temperature (°C) | 30 | 30 | 30 | 30 | 30 |
| Reaction time (hr) | 2 | 2 | 2 | 40 | 20 |
| pKa (calculated for pKa in MeOH) | 24.1 | 24.1 | 34.0 | 24.1 | 24.1 |
| Yield | 64% | 63% | 0% | 64% | 46% |
| Conversion rate | 100% | 98% | 5% | 98% | 98% |

| | Example 15 | Example 16 | Example 17 | Example 18 |
|---|---|---|---|---|
| Substrate | MFF | MFF | MFF | MFF |
| Solvents | THF | THF | Toluene | Dichloromethane |
| Bases | DBU | DBU | DBU | DBU |
| Nucleophilic Agents | MeOH | MeOH | MeOH | MeOH |
| Catalysts | CatE | CatE | CatE | CatE |
| Oxidizing Agents | Q1 | Q1 | Q9 | Q9 |
| Substrates (mmol) | 0.3 | 0.3 | 1 | 0.5 |
| Solvents (g) | 1 | 1 | 4.3 | 3.3 |
| Bases (mmol) | 0.09 | 0.09 | 1.1 | 0.55 |
| Catalysts (mmol) | 0.009 | 0.0009 | 0.15 | 0.15 |
| Nucleophilic agents (mmol) | 3 | 3 | 5 | 2.5 |
| Oxidizing agents (mmol) | 1 | 1 | 15 | 2.5 |
| Reaction temperature (°C) | 30 | 30 | 30 | 30 |
| Reaction time (hr) | 2 | 24 | 3(24) | 3(24) |
| pKa (calculated for pKa in MeOH) | 24.1 | 24.1 | 24.1 | 24.1 |
| Yield | 94% | 43% | 83% (96%) | 27% (59%) |
| Conversion rate | 100% | 75% | 100% | 100% |

In the above tables, pKa means pKa of the catalyst as calculated from free energy obtained by determining a stable structure of the catalytic molecule in methanol by the SMD method using Gaussian 16.

As is clear from the above results, using the catalyst with the pKa of 33.0 or less allowed the oxidation-reduction reaction to proceed between the alkylfuranaldehyde (MFF or the like) and the compound represented by A-OH (methanol or the like), thereby producing the alkylfurancarboxylic acid ester (comparison of Examples 1 to 3, compared with Comparative Example 1).

Further, by adding the oxidizing agent, the alkylfurancarboxylic acid ester could be produced at a high yield (Examples 4 to 12, compared with Comparative Example 2).

## Claims

1. A method for producing an alkylfurancarboxylic acid ester, the method comprising:
performing an oxidation-reduction reaction between an alkylfuranaldehyde and a compound represented by A-OH, where A is an organic group having 1 to 10 carbon atom(s), in the presence of a catalyst and a base,
wherein the catalyst is an N-heterocyclic carbene with a pKa of 33.0 or less, the pKa being calculated from a free energy obtained by determining a stable structure of a catalytic molecule in methanol by an SMD method using Gaussian 16.

2. The method for producing an alkylfurancarboxylic acid ester according to claim 1, wherein the method further comprises the performing the oxidation-reduction reaction in the presence of an oxidizing agent.

3. The method for producing an alkylfurancarboxylic acid ester according to claim 2, wherein the oxidizing agent is quinone.

4. The method for producing an alkylfurancarboxylic acid ester according to claim 2, wherein the oxidizing agent comprises at least one oxidizing agent represented by Formula (O): 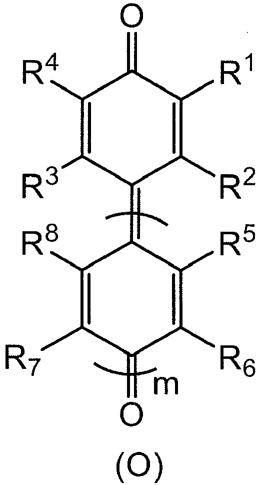 where each of R¹ to R⁸ independently represents a hydrogen atom, an alkyl group having 1 to 12 carbon atom(s), an alkenyl group having 2 to 12 carbon atoms, an alkynyl group having 2 to 12 carbon atoms, an alkoxy group having 1 to 12 carbon atom(s), an aryl group having 6 to 18 carbon atoms, an acyl group having 2 to 12 carbon atoms, or a hydroxy group; in a case where R¹ to R⁸ each are independently an alkyl group, an alkenyl group, an alkynyl group, an alkoxy group, an aryl group, or an acyl group, the groups of R¹ to R⁸ are each independently unsubstituted or substituted with a substituent boned to a carbon atom in the group, the substituent being an alkyl group having 1 to 6 carbon atom(s), an alkoxy group having 1 to 6 carbon atom(s), a hydroxy group, a halogen atom, a cyano group, or a nitro group; m is 1 or 0; and each pair of R¹ and R², R³ and R⁴, R⁵ and R⁶, and R⁷ and R⁸ may be independently bonded or condensed to each other to form a ring.

5. The method for producing an alkylfurancarboxylic acid ester according to claim 4, wherein the oxidizing agent represented by Formula (O) comprises at least one oxidizing agent represented by Formula (O-3): 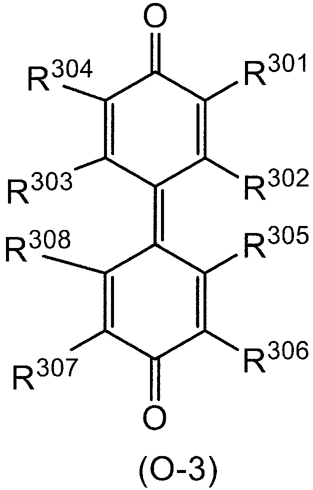 where each of R³⁰¹ to R³⁰⁸ in Formula (O-3) independently represents a group as defined for the R¹ in Formula (O).

6. The method for producing an alkylfurancarboxylic acid ester according to any one of claims 1 to 5, wherein the catalyst has a pKa of 16.0 or more.

7. The method for producing an alkylfurancarboxylic acid ester according to any one of claims 1 to 6, wherein the catalyst has a pKa of 30.0 or less.

8. The method for producing an alkylfurancarboxylic acid ester according to any one of claims 1 to 7, wherein the catalyst comprises at least one selected from the group consisting of triazolium, imidazolinium, imidazolium, and thiazolium.

9. The method for producing an alkylfurancarboxylic acid ester according to any one of claims 1 to 8, wherein the catalyst comprises at least one catalyst represented by Formula (C): 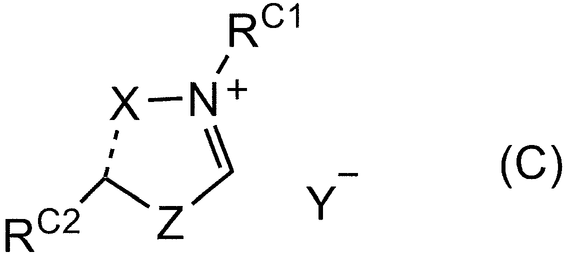 where each of R^{C1} and R^{C2} independently represents a hydrogen atom, an alkyl group having 1 to 12 carbon atom(s), an alkoxy group having 1 to 12 carbon atom(s), an aryl group having 6 to 18 carbon atoms, a heteroaryl group having 4 to 16 carbon atoms, a halogenated alkyl group having 1 to 12 carbon atom(s), a halogenated aryl group having 6 to 18 carbon atoms, an alkylaryl group having 7 to 20 carbon atoms, or an arylalkyl group having 7 to 20 carbon atoms; Z represents -S- or -NR^{c3}-; X is a methine group (=CR^{C5}-), a nitrogen atom (=N-) or a methylene group (-CR^{C6}₂-); in a case where X is a methine group or a nitrogen atom, the broken line in Formula (C) represents a double bond, and in a case where X is a methylene group, the broken line in Formula (C) represents a single bond; where each of R^{C3}, R^{C5}, and R^{C6} independently represents a hydrogen atom, an alkyl group having 1 to 12 carbon atom(s), an alkenyl group having 2 to 12 carbon atoms, an alkynyl group having 2 to 12 carbon atoms, an aryl group having 6 to 18 carbon atoms, an alkoxy group having 1 to 12 carbon atom(s), an acyl group having 2 to 12 carbon atoms, a hydroxy group, a carboxy group, or a halogen atom; Y⁻ represents a counter anion; and each pair of R^{C2} and R^{C3}, R^{C2} and R^{C5}, R^{C2} and R^{C6} may be independently bonded or condensed with each other to form a ring.

10. The method for producing an alkylfurancarboxylic acid ester according to claim 9, wherein the catalyst represented by Formula (C) includes at least one of catalysts represented by Formula (C2) to Formula (C6): 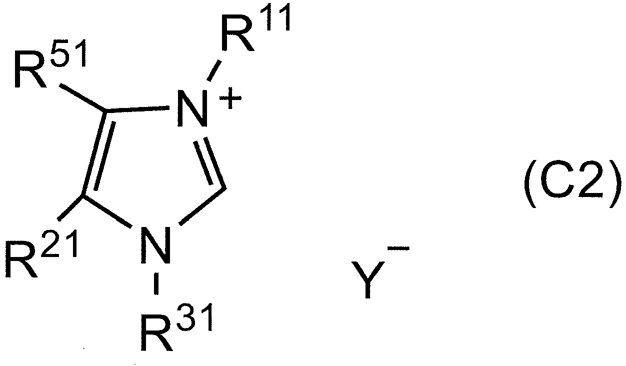
where, in Formula (C2), R¹¹ represents a group as defined for the R^{C1}, R²¹ represents a group as defined for the R^{C2}, R³¹ represents a group as defined for the R^{C3}, R⁵¹ represents a group as defined for the R^{C5}, Y⁻ represents a counter anion, and each pair of R²¹ and R³¹, R²¹ and R⁵¹, and R¹¹ and R⁵¹ may be independently bonded or condensed with each other to form a ring; 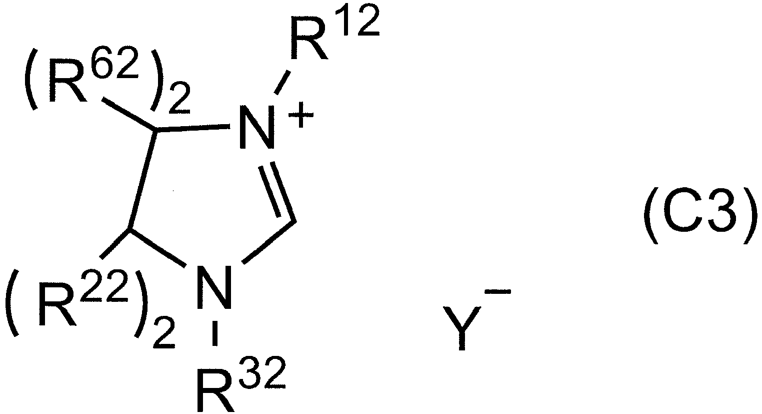
where, in Formula (C3), R¹² represents a group as defined for the R^{C1}, R²² represents a group as defined for the R^{C2}, R³² represents a group as defined for the R^{C3}, R⁶² represents a group as defined for the R^{C6}, Y⁻ represents a counter anion, and each pair of R²² and R³², R²² and R⁶², and R¹² and R⁶² may be independently bonded or condensed with each other to form a ring; 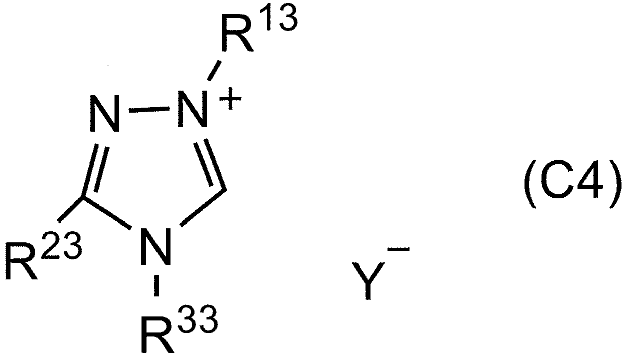
where, in Formula (C4), R¹³ represents a group as defined for the R^{C1}, R²³ represents a group as defined for the R^{C2}, R³³ represents a group as defined for the R^{C3}, Y⁻ represents a counter anion, and R²³ and R³³ may be bonded or condensed to each other to form a ring; 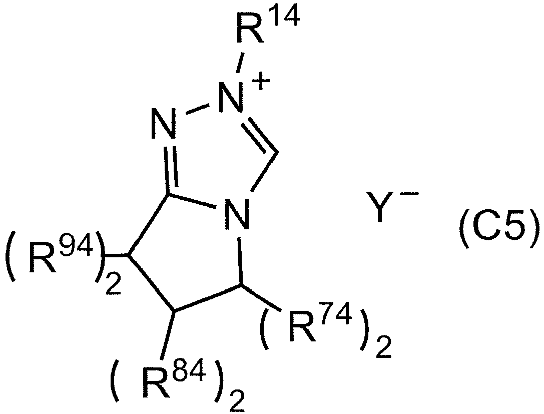
where, in Formula (C5), R¹⁴ represents a group as defined for the R^{C1}, R⁷⁴, R⁸⁴, and R⁹⁴ each independently represent a group as defined for the R^{C2}, Y⁻ represents a counter anion; 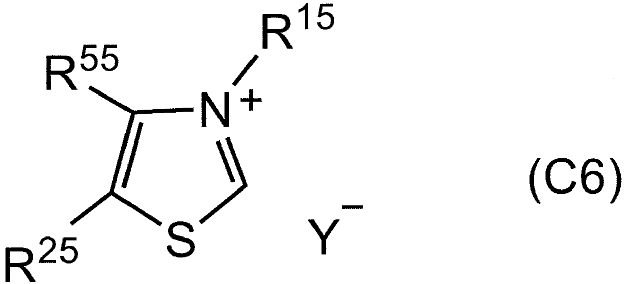
where, in Formula (C6), R¹⁵ represents a group as defined for the R^{C1}, R²⁵ represents a group as defined for the R^{C2}, R⁵⁵ represents a group as defined for the R^{C5}, Y⁻ represents a counter anion, and each pair of R¹⁵ and R⁵⁵, and R⁵⁵ and R²⁵ may be independently bonded or condensed to each other to form a ring.

11. The method for producing an alkylfurancarboxylic acid ester according to any one of claims 1 to 10, wherein the alkylfuranaldehyde is a compound represented by Formula (F1): 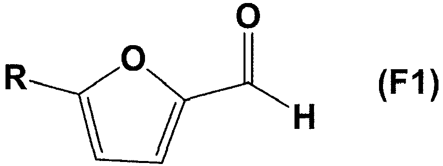 where, in Formula (F1), R is an alkyl group having from 1 to 10 carbon atom(s).

12. The method for producing an alkylfurancarboxylic acid ester according to any one of claims 1 to 11, wherein the alkylfurancarboxylic acid ester is a compound represented by Formula (F2): 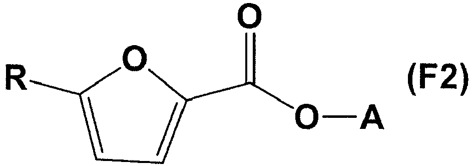 where, in Formula (F2), R is an alkyl group having 1 to 10 carbon atom(s), and A is an organic group having 1 to 10 carbon atom(s).

13. The method for producing an alkylfurancarboxylic acid ester according to any one of claims 1 to 12, wherein, in the compound represented by A-OH, A is an alkyl group having 1 to 5 carbon atom(s).
